# EUROPÄISCHE PATENTANMELDUNG

(11) **EP 0 540 895 A1**
(43) Veröffentlichungstag der Anmeldung: **12.05.1993**
(21) Anmeldenummer: 92117243.3
(22) Anmeldetag: 09.10.1992
(51) Int. Cl.: C07D 233/60, C07D 233/61, C08G 59/50

(54) **Verfahren zur Herstellung von latenten Härtern für Epoxidharze und deren Verwendung**

(30) Priorität: 15.10.1991 DE 4134081
(71) Anmelder: Witco GmbH, D-59180 Bergkamen (DE)
(72) Erfinder: Burba, Christian, Dr. Dipl.-Chem., W-4715 Ascheberg-Herbern (DE); Mrotzek, Werner, Dr. Dipl.-Ing., W-4600 Dortmund 1 (DE); Volle, Jörg, W-4714 Selm-Bork (DE)

(57) **Zusammenfassung**

Die Erfindung betrifft ein Verfahren zur Herstellung von latenten Härtern für Epoxidharze auf Basis von Amid- und gegebenenfalls Amingruppen enthaltenden Imidazol- oder N-Aminoalkylimidazol-Acrylsäure-Reaktionsprodukten und deren Verwendung zur Herstellung von faserverstärkten Platinen für die Elektrotechnik.

## Beschreibung

Die Erfindung betrifft ein Verfahren zur Herstellung von latenten Härtern für Epoxidharze auf Basis von Amid- und gegebenenfalls Amingruppen enthaltenden Imidazol- oder N-Aminoalkylimidazol-Acrylsäure-Reaktionsprodukten und deren Verwendung zur Herstellung von faserverstärkten Platinen für die Elektrotechnik.

In der modernen Technik werden seit geraumer Zeit Bauteile verwendet, in welchen anstelle von Verdrahtungen elektrische Schaltkreise in Form von aufgedampften Leiterbahnen Verwendung finden (PCB).

Hierbei werden auf isolierende Trägermaterialien dünne elektrisch leitfähige Schichten in verschiedenen Verfahrensweisen aufgebracht. Für diese Anwendung werden an die Homogenität der jeweiligen Trägermaterialien hohe Anforderungen gestellt, welche sich im Zuge der fortschreitenden Miniaturisierung noch laufend verschärfen. Jede Inhomogenität in Größenordnung der geometrischen Abmessungen der Leiterbahnen (Breite und Stärke) kann hier zu tiefgreifenden funktionellen Störungen führen.

Als Trägermaterialien finden zur Zeit überwiegend faserverstärkte Werkstoffe Verwendung, in welchen als Bindemittel härtbare Mischungen aus Epoxidharzen und aminischen Härtungsmitteln eingesetzt werden.

In der Regel werden hierbei in erster Stufe aus den Verstärkungsmaterialien und dem Bindemittel sogenannte Prepregs hergestellt, welche dann zwischengelagert werden können, ehe sie unter Anwendung von Druck und erhöhten Temperaturen ausgehärtet werden.

Bei den Prepregs befindet sich das Bindemittel im sogenannten B-Zustand, das heißt, es wurde bis zu einem gewissen Grade angehärtet. Es ist daher fest, aber noch weitgehend in Lösungsmitteln löslich und auch schmelzbar.

Zur Erzielung dieses Zustandes werden an das Härtungsmittel besondere Anforderungen gestellt:
Es soll bei möglichst geringem Energieaufwand (Temperatur, Zeit) die Erzielung des B-Zustandes ermöglichen, nach Abkühlung auf Raumtemperatur diesen Zustand jedoch ohne weitere Veränderung über einen langen Zeitraum erhalten.

Die Aushärtung soll wiederum schnell und vollständig bei möglichst niederen Temperaturen innerhalb kurzer Zeit und ohne große Exothermie erfolgen.

Hierbei müssen die Endprodukte natürlich die geforderten physikalischen und mechanischen Anforderungen der Praxis erfüllen.

Mit Dicyandiamid gehärtete Epoxidharze erfüllen hinsichtlich physikalischer und mechanischer Eigenschaften sowie Lagerungs- und Härtungsverhalten weitgehend diese Forderungen, so daß es zur Zeit vorwiegend, gegebenenfalls unter Mitverwendung von Beschleunigern, eingesetzt wird.

Das vorteilhafte Lagerungs- und Härtungsverhalten ist auf die bei Raumtemperatur weitgehende Unlöslichkeit des Dicyandiamids in den üblichen Epoxidharzen zurückzuführen.

Bei Einsatz von festem kristallinen Dicyandiamid werden in den gehärteten Substraten Inhomogenitäten festgestellt, welche auf nicht gelösten und nicht umgesetzten Partikeln beruhen.

Bei Verwendung von Dicyandiamidlösungen können zwar homogene Substrate hergestellt werden, jedoch bringt die Verwendung von Lösungsmitteln andere Probleme mit sich.

Dicyandiamid ist nur in wenigen Lösungsmitteln wie insbesondere Dimethylformamid oder Methylglykol in ausreichenden Mengen löslich. Diese Lösungsmittel sind jedoch toxikologisch bedenklich und bringen sowohl bei der Herstellung der Prepregs, das heißt bei Imprägnierung der Verstärkungsmaterialien und Überführung in den B-Zustand, als auch bei der Entsorgung Probleme mit sich.

Aufgrund der Schwerlöslichkeit des Dicyandiamids müssen größere Mengen an Lösungsmittel verwendet werden, welche die Tränkviskosität so beeinflussen, daß der Bindemittelgehalt auf den Verstärkungsmaterialien nicht beliebig gewählt werden kann.

Da diese Lösungsmittel bei der Härtung nicht vollständig entfernt werden können, besteht bei einer thermischen Belastung der Bauteile in den jeweiligen Geräten weiterhin die Gefahr, daß die Lösungsmittel vor Ort unkontrolliert an die Umgebungsluft abgegeben werden.

Aufgabe der vorliegenden Erfindung war es daher, diese Nachteile zu überwinden und ein Verfahren zur Herstellung von faserverstärkten Trägermaterialien zu finden, welche in der Elektrotechnik, insbesondere der Elektronik und Mikroelektronik, zur Herstellung von Platinen (PCB) verwendet werden können.

Diese Aufgabe wird erfindungsgemäß gelöst durch Mitverwendung einer härtbaren Mischung aus Epoxidharzen und latenten Amid- und gegebenenfalls Amingruppen enthaltenden Härtungsmitteln auf Basis von Imidazol- und/oder N-Aminoalkylimidazol-Acrylsäure-Reaktionsprodukten.

Gegenstand der Erfindung sind Amid- und gegebenenfalls Amingruppen enthaltende Verbindungen auf Basis von Imidazol- und/oder N-Alkylimidazol-Acrylsäure-Reaktionsprodukten, herstellbar in erster Stufe durch
A) Addition von Acrylsäure(estern) an gegebenenfalls substituierte Imidazole und/oder N-Aminoalkylimidazole in ca. equivalenten Mengen, bezogen auf reaktive Aminwasserstoffatome und Doppelbindungen und
weitere Umsetzung dieser Addukte in zweiter Stufe mit
B) Polyaminen und/oder Aminoalkoholen, welche im Durchschnitt zwei oder mehr reaktive Wasserstoffatome im Molekül haben, unter Kondensationsbedingungen zu Polyamiden und/oder Polyaminoamiden bzw. Polyamidoalkoholen, wobei
gegebenenfalls in einer dritten Stufe
C) die Reaktionsprodukte AB) mit ca. equivalenten Mengen an Mono- und/oder Polyglycidylverbindungen, Mono- und/oder Polyacrylaten, Mono- und/oder Polycarbonsäuren verlängert werden.

Ein weiterer Gegenstand der Erfindung ist dadurch gekennzeichnet, daß als Härtungsmittel Verbindungen gemäß den Ansprüchen 1 - 3 mitverwendet werden.

Ein weiterer Gegenstand der Erfindung ist dadurch gekennzeichnet, daß die Härtungsmittel gemäß den Ansprüchen 1 und 3 mitverwendet werden.

Ein weiterer Gegenstand der Erfindung ist dadurch gekennzeichnet, daß als Epoxidharze gemäß Anspruch 1 c) Glycidylether auf Basis von Bisphenol A, Bisphenol F und Novolaken mit Epoxidwerten von 0,18 - 0,6, insbesondere 0,39 - 0,55, mitverwendet werden. Vorzugsweise werden handelsübliche halogenierte, insbesondere bromierte Epoxidharze mit ca. 18 Gew.-% Brom auf Basis der oben angegebenen Rohstoffe verwendet.

Die Herstellung der faserverstärkten Basismaterialien für Leiterplatten erfolgt wegen der zum Teil recht hohen Viskosität der eingesetzten Epoxidharze überwiegend im Zweistufenverfahren, denn nur dabei kann die Viskosität des Bindemittelsystems durch Zugabe von Lösungsmitteln in weiten Bereichen variiert werden.

Bei diesem Verfahren werden aus den Verstärkungsmaterialien und der härtbaren Mischung zunächst sogenannte Prepregs hergestellt, die dann in einem zeitlich getrennten 2. Verfahrensschritt zu Fertigteilen weiterverarbeitet werden.

Die Herstellung der Prepregs erfolgt normalerweise in einem kontinuierlichen Prozeß, in dem die Verstärkungsmaterialien durch ein Imprägnierbad des einzusetzenden Harz-Härter-Gemisches geleitet werden. Die Steuerung der auf einer bestimmten Trägermaterialbahn aufzubringenden Imprägniermittelmenge erfolgt dabei außer über die Viskosität des Imprägniermittels über nachgeschaltete Abquetschwalzen.

Bei lösungsmittelhaltigen Systemen wird nach dem Imprägnierprozeß durch Wärmezufuhr das in der Imprägnierlösung enthaltene Lösungsmittel verdampft und gleichzeitig das Harzsystem vom A- in den B-Zustand überführt. Aus den mit flüssigem bis stark klebrigem Imprägniermittel getränkten Verstärkungsmaterialien wird je nach Verfahrensbedingungen und verwendetem Harzsystem ein schwach klebriger bis fast trockener Prepreg. Bei diesem Verfahrensschritt ist es wichtig, daß einerseits das Lösungsmittel der Imprägniermischung vollständig entzogen wird, andererseits aber der für die Prepreg-Härtung im 2. Verfahrensschritt notwendige latente Härter noch nicht anspringt, um ein ungewolltes Ausreagieren der imprägnierten Verstärkungsmaterialien zu verhindern.

Bei lösungsmittelfreien Systemen erfolgt abhängig von der chemischen Zusammensetzung des Harzsystems nach der Imprägnierung entweder ebenfalls eine kurze Wärmebehandlung des Materials, um das Imprägniermittel in den B-Zustand zu überführen, oder die Verstärkungsstoffe werden unmittelbar nach der Imprägnierung unter Verzicht auf eine gesonderte Wärmebehandlung beidseitig mit Trennfolien kaschiert und einer systemadäquaten Zwischenlagerung zugeführt. Im Verlaufe dieser Zwischenlagerung tritt entweder ein allmählicher Übergang des Harzsystems in den B-Zustand ein oder das Imprägniermittel wird unter weitgehendem Verzicht auf chemische Veränderungen allein durch physikalische Effekte auf den Trägermaterialien fixiert.

Die so erhaltenen Prepregs lassen sich als Rollen zwischenlagern und transportieren, ehe sie dem jeweiligen Anwendungsfall entsprechend zurechtgeschnitten und in Bauteildicke übereinander geschichtet werden. Durch gleichzeitige Druck- und Temperatureinwirkung wird der Prepregstapel zu einem hochfesten Formteil ausgehärtet, wobei die noch niedermolekularen, fließfähigen Harze in den hochmolekularen C-Zustand des Duromers übergehen.

Während beim Einstufenverfahren nur lange offene Zeiten und kurze Aushärtungszeiten bei niedrigen Härtungstemperaturen gefordert werden, kommt beim Zweistufenverfahren als weiteres Kriterium noch eine möglichst lange Lagerstabilität der Prepregs hinzu. Dabei werden Lagerungstemperaturen unterhalb der Raumtemperatur immer weniger akzeptiert.

Von Bedeutung ist weiterhin, daß je nach Herstellungsverfahren der Prepregs die Materialvikosität der gebrauchsfertigen härtbaren Mischung über einen möglichst langen Zeitraum weitgehend unverändert bleibt. Speziell bei Verwendung eines großvolumigen Tränkbades ist dies für die Erzielung eines konstanten Harzauftrages und eines gleichbleibenden B-Zustandes erforderlich, da zum einen die Bedingungen der Produktion nicht laufend den sich ändernden Verhältnissen in der härtbaren Mischung angepaßt werden können und zum anderen dadurch auch die physikalischen Eigenschaften des ausgehärteten Endproduktes negativ beeinflußt werden.

Angestrebt wird in der Praxis eine härtbare Mischung, welche im Imprägnierbad auch über längere Zeit viskositätsmäßig konstant bleibt, bei geringen Temperaturen in kurzer Zeit zum B-Zustand reagiert und dann als Prepreg bei Raumtemperatur ohne chemische Veränderungen lange gelagert werden kann.

Die nachfolgende Prepreg-Aushärtung soll bei möglichst tiefen Temperaturen innerhalb kurzer Zeit erfolgen, das Temperaturmaximum der exothermen Reaktion auch bei größeren Schichtdicken auf einem niedrigen Niveau verbleiben und das physikalische Eigenschaftsniveau der Endprodukte den Erfordernissen der Praxis angepaßt sein.

Das in härtbaren Mischungen auf Basis von Epoxidharzen seit langem als Härter verwendete Dicyandiamid wird zur Erzielung der angestrebten Eigenschaften in der Regel mit Co-Härtungsmitteln und/oder Beschleunigern kombiniert. Aus der Literatur sind auf diesem Gebiet daher eine Vielzahl von Vorschlägen bekannt geworden.

Zur Erzielung homogener Substrate ist es jedoch erforderlich, das Dicyandiamid in gelöster Form einzusetzen, da ansonsten in den Substraten kristalline, nicht umgesetzte Partikel verbleiben.

Die wenigen Lösungsmittel, in denen Dicyandiamid in ausreichenden Mengen löslich ist, sind jedoch physiologisch bedenklich und bereiten verarbeitungstechnische Probleme.

Die erfindungsgemäß mitverwendeten Härtungsmittel sind dagegen in allen gängigen, auch leicht flüchtigen und physiologisch unbedenklichen Lösungsmitteln in ausreichender Menge löslich. Dadurch kann sowohl die Tränkviskosität der härtbaren Mischung den Erfordernissen der Praxis angepaßt werden als auch das Lösungsmittel bei der Überführung in den B-Zustand problemlos praktisch vollständig entfernt werden.

Als erfindungsgemäß mitverwendbare Lösungsmittel kommen die auf diesem Gebiet gebräuchlichen Lösungsmittel wie Alkohole, aliphatische und aromatische Kohlenwasserstoffe und Glykolether sowie Mischungen wie z. B. Alkohole/Ketone in Frage. Erfindungsgemäß bevorzugt werden kurzkettige Alkohole wie Ethanol, Propanol oder deren Mischungen.

Die erfindungsgemäß mitverwendeten Verbindungen gemäß Anspruch 1 wirken sowohl als Härtungsmittel als auch als Beschleuniger, so daß auf die mögliche Mitverwendung üblicher Beschleuniger und Härtungsmittel verzichtet werden kann. Andererseits können die erfindungsgemäßen Verbindungen mit Erfolg als Beschleuniger in handelsüblichen Härtungsmitteln, wie beispielsweise Dicyandiamid, eingesetzt werden.

Die erfindungsgemäßen Härtungsmittel sind nach an sich bekannten Einzelverfahren herstellbar durch Addition von Acrylsäure oder Acrylsäurederivaten der allgemeinen Formel I 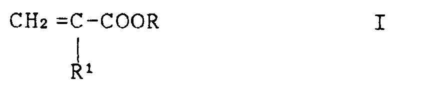
mit R = H oder kurzkettiger Alkylrest mit 1 - 4 C-Atomen, insbesondere Methyl- oder Ethylrest und
R¹ = H oder CH₃- mit Imidazolen der allgemeinen Formel II 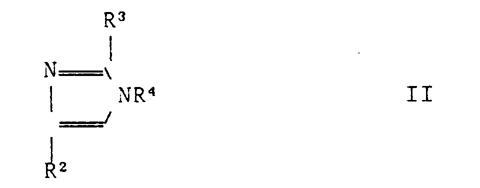
worin R² und R³ unabhängig voneinander H, CH₃-, C₂H₅-, Phenyl-Reste und R⁴ H oder der Rest -(CH₂)ₐ-NH₂ mit a = 2 oder 3 sein kann, wie insbesondere die Verbindungen Imidazol, 2-Methylimidazol, 2-Ethyl-4-methyl-imidazol, 1-(3-Aminopropyl)imidazol.

Sowohl die Acrylate als auch die Imidazole können jeweils allein oder in Mischung miteinander eingesetzt werden. Im allgemeinen wird dabei das Imidazol, gegebenenfalls unter Mitverwendung eines Lösungsmittels, auf 30 - 90 °C, vorzugsweise 50 - 70 °C, erwärmt und bei dieser Temperatur das Acrylat langsam zugetropft. Die Mengenverhältnisse, bezogen auf reaktive Aminwasserstoffatome und Doppelbindungen, sind dabei equivalent.

Diese Additionsprodukte, welche idealisiert die allgemeine Formel III 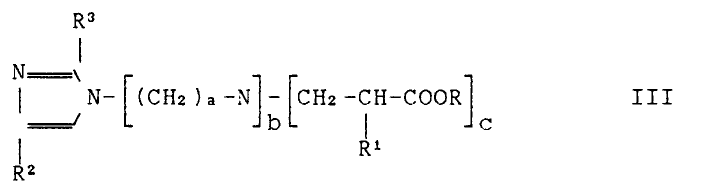
aufweisen, worin R, R¹, R², R³ und a die oben angegebene Bedeutung haben und b = 0 oder 1 und c = 1 oder 2 sein kann, wobei gilt, daß nur, wenn b 1 ist, c = 1 oder 2 sein kann und, wenn b = 0 ist, c = 1 ist, werden in einer zweiten Stufe mit Polyaminen bzw. Alkanolaminen zu den entsprechenden Amidgruppen und gegebenenfalls Amingruppen und/oder Hydroxylgruppen enthaltenden Verbindungen umgesetzt.

Als Polyamine bzw. Alkanolamine sind erfindungsgemäß alle Amine und Aminoalkohole einsetzbar, welche zwei oder mehr reaktive Wasserstoffatome pro Molekül enthalten und welche unter den angegebenen Bedingungen mit der Säuregruppe der Additionsverbindungen der Formel III reagieren. Dies sind mono- und/oder polyfunktionelle, aliphatische, cycloaliphatische, heterocyclische und gegebenenfalls aromatische oder araliphatische Amine bzw. Aminoalkohole, welche gegebenenfalls Heteroatome wie insbesondere Sauerstoff und/oder Stickstoff enthalten und gegebenenfalls verzweigt sein können. Erfindungsgemäß bevorzugt werden heterocyclische Amine wie N-Aminoethylpiperazin, aliphatische Amine wie Diethylentriamin und Aminoalkohole wie Diglykolamin.

Die Kondensationsreaktion wird dabei nach den an sich bekannten Methoden durchgeführt. Im allgemeinen wird dabei so verfahren, daß zu dem vorgelegten Amin bzw. Aminoalkohol die Verbindungen der allgemeinen Formel III zugegeben und unter Rühren, gegebenenfalls in Gegenwart eines Schleppmittels, auf 180 - 220 °C aufgeheizt werden, bei dieser Temperatur die Hauptmenge Reaktionswasser ausgekreist und die Reaktion bei Unterdruck von ca. 10 - 50 mbar beendet wird.

Die Molverhältnisse können in Abhängigkeit von den Einsatz- und den gewünschten Reaktionsprodukten ca. equivalent sein oder so gewählt werden, daß freie Aminwasserstoffatome im Reaktionsprodukt verbleiben.

Reaktionsprodukte mit freien Aminwasserstoffatomen können dann in einer weiteren Stufe mit reaktiven mono- oder polyfunktionellen Verbindungen verlängert werden. Als reaktive Verbindungen können prinzipiell alle Verbindungen eingesetzt werden, welche mit den Aminwasserstoffatomen bei Raumtemperatur oder unter Kondensationsbedingungen reagieren. Erfindungsgemäß bevorzugt werden Mono- und/oder Diglycidylverbindungen, Mono- und/oder Diacrylate, Mono- und/oder Dicarbonsäuren. Diese Verbindungen können allein oder in Mischung miteinander oder untereinander mit den Amingruppen enthaltenden Verbindungen umgesetzt werden. Die Molverhältnisse werden dabei vorzugsweise so gewählt, daß keine Überschüsse der einen oder anderen Komponente im Reaktionsgemisch verbleiben, insbesondere aber keine freien Aminwasserstoffatome.

Als erfindungsgemäß mitverwendbare Mono- und/oder Diglycidylverbindungen werden bevorzugt eingesetzt Butandioldiglycidylether und Hexandioldiglycidylether.

Als erfindungsgemäß mitverwendbare Mono- und/oder Diacrylate werden bevorzugt eingesetzt Butandiol-Diacrylat und Hexandiol-Diacrylat.

Als Carbonsäuren sind aliphatische, cycloaliphatische, aromatische und araliphatische mono- und polyfunktionelle Verbindungen, welche unter den angegebenen Bedingungen mit den Aminen bzw. Aminoalkoholen reagieren, einsetzbar. Erfindungsgemäß bevorzugt werden aliphatische Dicarbonsäuren wie Adipinsäure, Azelainsäure und dimerisierte Fettsäuren, welche aus den ungesättigten monomeren einbasischen Säuren mit 12 - 18 C-Atomen nach den bekannten Verfahren hergestellt werden und Dimergehalte von ca. 65 Gew.-% und mehr aufweisen können.

Als erfindungsgemäß mitverwendbare Mono- und/oder Dicarbonsäuren werden bevorzugt eingesetzt Adipinsäure, Azelainsäure und dimerisierte Fettsäuren.

Die Menge an Härtungsmitteln bzw. Härtungsbeschleunigern kann innerhalb weiter Grenzen variiert werden. Sie wird bestimmt durch den beabsichtigten Anwendungszweck und die gegebenenfalls dadurch vorgegebenen Härtungsbedingungen. Erfindungsgemäß werden Mengen im Bereich von 4 bis 20, vorzugsweise 5 bis 10 Gewichtsteilen, bezogen auf 100 Gewichtsteile Epoxidverbindung, angewandt.

Die zur Herstellung der härtbaren Mischung mitverwendeten Epoxidharze sind Glycidylester und -ether mit zwei oder mehr Epoxidgruppen pro Molekül wie vorzugsweise die Glycidylether auf Basis von ein- oder mehrwertigen Phenolen. Erfindungsgemäß bevorzugt werden Glycidylether von 2,2-Bis(4-hydroxyphenyl)-propan (Bisphenol A) mit Epoxidwerten von 0,2 - 0,6, insbesondere die bei Raumtemperatur halbfesten hochviskosen bis mittelviskosen Verbindungen mit Epoxidwerten um 0,39 bis 0,55. Daneben haben sich auch die Glycidylether auf Basis von Bisphenol F und die Novolake als vorteilhaft erwiesen.

Vorzugsweise werden handelsübliche halogenierte, vorzugsweise bromierte Epoxidharze mit den bekannten Bromgehalten auf Basis der oben angeführten Harze eingesetzt.

Zur Modifizierung der Eigenschaften des Endprodukts können neben anderen Epoxidharzen auch Modifizierungs- oder Hilfsstoffe mitverwendet werden wie Phenolharze, Melaminharze, Silikonharze.

Zur Einstellung der gewünschten Viskosität können unterschiedlich viskose Harze oder Verdünnungsmittel mitverwendet werden, aber auch die üblichen Lösungsmittel wie Dimethylformamid, Aceton, Methylethylketon, Methylglykol, Propylenglykolmonomethylether oder deren Mischungen eingesetzt werden.

Zur Herstellung der Prepregs werden organische und anorganische Fasern, Vliese und Gewebe, insbesondere aber Glas, mitverwendet.

Die Herstellung der lösungsmittelhaltigen Prepregs geschieht nach der an sich bekannte Methode, bei der die Trägermaterialien in einem Imprägnierbad mit der reaktiven Harzmischung getränkt und nach dem Abquetschen der überschüssigen Harzmenge kontinuierlich unter Energiezufuhr (meist Wärme) bei gleichzeitigem Entzug des Lösungsmittels vom A- in den B-Zustand überführt werden. Je nach gewünschter Prepreg-Konsistenz (klebrig bis fest) werden diese anschließend beidseitig mit einer Trennfolie versehen und für die Lagerung und den Transport aufgerollt. Die Weiterverarbeitung erfolgt durch Zuschneiden und Zusammenlegen der einzelnen Prepreg-Lagen zu einem Stapel, aus dem durch formgebende Maßnahmen unter gleichzeitiger Wärmezufuhr ein hochvernetztes Werkstück entsteht.

Die erfindungsgemäßen Härtungsmittel können außerdem mit Erfolg in lösungsmittelfreien Prepregs auf Basis von Epoxidharzen und gegebenenfalls üblichen Härtungsmitteln verwendet werden. Hierbei werden die Trägermaterialien bei gegebenenfalls erhöhter Temperatur nach an sich bekannten Verfahren mit den Bindemittelsystemen getränkt und systemadäquat gelagert, ehe sie wie die Lösungsmittel enthaltenden Systeme weiterverarbeitet werden.

Weitere Beispiele für lösungsmittelfreie Systeme sind unter anderem heißhärtende Einkomponentenklebstoffe z. B. für die Verklebung von Karosserieteilen in der Automobilindustrie und Epoxidharz-Beschichtungen, die entweder naß oder als Pulver aufgebracht werden.

### Herstellung der erfindungsgemäßen Härtungsmittel

1. Umsetzung des Imidazol-Acrylsäure-Adduktes mit N-Aminoethylpiperazin
   1.068,6 g Imidazol werden bei Raumtemperatur (RT) in 3.300 g destilliertem Wasser gelöst. Man erwärmt auf ca. 40 - 50 °C und tropft 1.131,4 g Acrylsäure zu. Nach Beendigung der Zugabe läßt man 2 h bei 50 °C nachreagieren.
   Das Reaktionsprodukt (40 %ige Lösung in Wasser) wird im Vakuum vom Wasser befreit und bei ca. 120 °C mit 2.026 g N-Aminoethylpiperazin langsam versetzt. Man erhitzt auf ca. 180 °C und destilliert über einen Dephlegmator die theoretisch mögliche Wassermenge ab.
   Nach Abkühlen erhält man ein pastöses, hochviskoses Produkt.
   - Viskosität bei 60 °C:: ca. 50,0 Pa·s.
   - AZ:: ca. 670
   - SZ:: 1,3
   Es entspricht dem über eine äquivalente Menge Acrylsäuremethylester (anstelle Acrylsäure) hergestellten Produkt.
2. Umsetzung des Aminopropylimidazol-Acrylsäure-Adduktes mit N-Aminoethylpiperazin
   500 g Aminopropylimidazol werden in 360 g destilliertem Wasser vorgelegt. Man erwärmt auf 50 - 60 °C und tropft 576 g Acrylsäure zu. Nach Beendigung der Zugabe läßt man 1 h bei 60 °C nachreagieren. Das Reaktionsprodukt ist 75 %ig in Wasser.
   320 g dieses Produktes werden mit 248 g (ca. 10 % Überschuß) N-Aminoethylpiperazin vorsichtig zusammengegeben. Beim Erhitzen auf ca. 120 °C destilliert die eingesetzte Wassermenge ab und ab 180 °C destillieren ca. 32 g durch Amidbildung entstandenes Wasser ab. Man heizt auf ca. 210 °C auf, hält ca. 2 h bei dieser Temperatur und zieht im Vakuum von 10 mbar überschüssiges Amin ab. Das Produkt hat folgende Kennzahlen:
   - AZ:: 599
   - SZ:: 0,5
   und eine schmalzartige Konsistenz.
3. Umsetzung 2-Methylimidazol-Acrylsäure-Adduktes mit Diethylentriamin
   164 g 2-Methylimidazol werden bei ca. 40 °C in 308 g destilliertem Wasser gelöst und bei dieser Temperatur langsam mit 144 g Acrylsäure versetzt. Nach Beendigung der Zugabe wird 2 h bei 50 °C nachgerührt. Die erhaltene 50 %ige Lösung wird für weitere Umsetzungen benutzt.
   51,5 g Diethylentriamin werden mit 308 g der obengenannten 2-Methylimidazol/Wasser-Lösung langsam versetzt. Beim Erhitzen destilliert die eingesetzte Wassermenge ab. Beim weiteren Aufheizen auf ca. 180 °C kann ca. 1 Mol Reaktionswasser abdestilliert werden. Das Produkt hat folgende Kennzahlen:
   - AZ:: 429
   - SZ:: 8,2.
4. Umsetzung des Imidazol-Acrylsäure-Adduktes gemäß Beispiel 1 mit Diglykolamin
   350 g des Imidazol-Acrylsäureadduktes (40 %ig in Wasser) gemäß Beispiel 1 werden zu 115 g (10 % Überschuß) Diglykolamin langsam zugegeben. Beim Erhitzen destilliert die eingesetzte Wassermenge ab. Beim weiteren Erhitzen entsteht ab 160 °C Reaktionswasser. Man hält 2 h bei 190 °C und entfernt schließlich das restliche Wasser und das überschüssige Amin im Vakuum. Das Reaktionsprodukt hat folgende Kennzahlen:
   - AZ:: 261
   - OH-Z:: 257
   - SZ:: 5,9.
5. Umsetzung des Reaktionsproduktes aus Imidazol/Acrylsäure/N-Aminoethylpiperazin gemäß Beispiel 1 mit Hexandiol-Diglycidylether
   125,5 g des obengenannten Adduktes gemäß Beispiel 1 werden vorgelegt, auf 100 - 120 °C erhitzt und mit 79,5 g Hexandioldiglycidylether (Ep-Wert 0,63) langsam versetzt. Man rührt 2 h bei 120 °C nach.
   - AZ:: 410
6. Umsetzung des Reaktionsproduktes aus Imidazol/Acrylsäure/N-Aminoethylpiperazin gemäß Beispiel 1 mit Hexandiol-Diacrylat
   125,5 g des obengenannten Adduktes gemäß Beispiel 1 werden vorgelegt, auf 100 - 120 °C erhitzt und mit 56,5 g Hexandiol-Diacrylat langsam versetzt. Man rührt 2 h bei 120 °C nach.
   - AZ:: 460
7. Umsetzung des 2-Methylimidazol-Acrylsäure-Adduktes und der dimeren Fettsäure mit Diethylentriamin
   308 g 2-Methylimidazol/Acrylsäure-Addukt (50 %ig in Wasser) werden vorgelegt und mit 51,5 g Diethylentriamin gemäß Beispiel 3 zur Umsetzung gebracht und anschließend bei 180 °C mit 290 g (Säure-Überschuß) dimerer Fettsäure (Dimergehalt 96 %, SZ: 193) versetzt. Man erhitzt 2 h bei 230 °C, bis alles Reaktionswasser abdestilliert ist.
   - AZ:: 133
   - SZ:: 55
   Die in den obengenannten Beispielen angegebenen Analysenwerte AZ = Aminzahl, SZ = Säurezahl, OH-Z = Hydroxylzahl, werden nach den auf diesem Gebiet üblichen Methoden bestimmt und werden angegeben in mg KOH/g Substanz.

### Ermittlung der Härtungsmitteleinflüsse in Verbindung mit bromierten Harzen

### Beispiel 1

100 g eines in 25 g Methylethylketon (MEK) gelösten bromierten Epoxidharzes (Epoxidwert ca. 0,18, Bromgehalt ca. 18 %) werden mit 10 g einer 50 %igen Härterlösung aus dem erfindungsgemäßen Reaktionsprodukt und Ethanol vermischt und zur Prepreg-Fertigung eingesetzt. Die Aufbereitung der Härterlösung erfolgt bei Raumtemperatur, wobei deren Konzentration grundsätzlich beliebig gewählt werden kann.

Zur Prepregherstellung im Labormaßstab wird zunächst ein ca. 0,1 m² großes Glasfilamentgewebe mit der Harz-Härter-Lösung getränkt. Daran anschließend erfolgt eine Wärmebehandlung des derart imprägnierten Verstärkungsmaterials von 2 min bei 120 °C in einem Umlufttrockenschrank. Während dieses Prepregvorgangs wird zum einen das Lösemittel aus dem Imprägniermittel entfernt, und zum zweiten werden die Reaktionskomponenten von dem A- in den B-Zustand übergeführt. Es resultieren nahezu trockene, flexible Prepregs, die nach der Abkühlung bei Raumtemperatur bis zur Weiterverarbeitung mehrere Wochen gelagert werden können.

Die Prepregweiterverarbeitung zu ca. 1 mm dicken Laminaten erfolgt in einer Stunde bei 150 °C mit einem Preßdruck von ca. 1 bar. Das derart ausgehärtete Endprodukt zeigt keinerlei Mängel bezüglich der Haftung der einzelnen Prepreglagen.

Zur Bestimmung der thermischen Beständigkeit des Matrixmaterials werden anschließend aus den Laminaten 100 x 10 mm² große Probekörper und im Torsionsschwingversuch nach DIN 53455 untersucht. Die Ergebnisse dieser Prüfung sind für verschiedene Mischungsverhältnisse in Tabelle 1 wiedergegeben.

Die weiteren ebenfalls in Tabelle 1 aufgeführten TG-Werte der Beispiele 2 - 7 werden analog Beispiel 1 mit dem dort angeführten Epoxidharz ermittelt. Als Vergleichsgröße wird zusätzlich der TG eines derzeit häufig benutzten Matrixmaterials mit Dicyandiamid als Härter und N-Methylimidazol als Beschleuniger mit in die Tabelle aufgenommen (Mischungsverhältnis bromiertes Epoxidharz : Dicyandiamid : N-Methylimidazol = 100 : 5 : 0,8.

**Tabelle 1**

| Thermische Beständigkeit der Laminate | | | | | |
|---|---|---|---|---|---|
| Beispiel (Härtungsmittel) | Mischungsverhältnis Harz : Härter ter | TG in °C nach Prepreglagerung von | | | |
| | | 1 Tag | 4 Wochen | 12 Wochen | 26 Wochen |
| 1 | 100 : 5 | 127 | 116 | 116 | 115 |
| | 100 : 10 | 125 | | | |
| 2 | 100 : 5 | 124 | 120 | 118 | |
| | 100 : 10 | 129 | 121 | 120 | |
| 3 | 100 : 5 | 145 | 131 | 127 | |
| | 100 : 10 | 151 | 136 | | |
| 4 | 100 : 5 | 126 | 122 | 119 | |
| | 100 : 10 | 123 | | | |
| 5 | 100 : 5 | 124 | 118 | 117 | 117 |
| | 100 : 10 | 118 | | | |
| 6 | 100 : 5 | 128 | 115 | 110 | 110 |
| | 100 : 10 | 130 | | | |
| 7 | 100 : 5 | 119 | 119 | | |
| | 100 : 10 | 125 | 123 | | |
| Vergleich | 100 : 5 : 0,8 | 118 | | | |

### Ermittlung der Härtungsmitteleinflüsse in Verbindung mit BPA-Basisharzen

Zur Ermittlung der Materialeigenschaften bei Verwendung der ausgewählten Härtungsmittel werden zur Vermeidung verfälschender Einflüsse von Verstärkungs- und Zusatzmaterialien die Mischungen, bestehend allein aus Epoxidharz und Härtungsmittel, ausgehärtet und abgeprüft.

In den in der Tabelle 2 aufgelisteten Beispielen wird als Epoxidharz ein Glycidylether auf Basis von Bisphenol A mit einem Epoxidwert von 0,53 verwendet.

Zur Herstellung der Prüfkörper werden jeweils 100 g des Epoxidharzes mit der in der Tabelle 2 genannten Menge Härtungsmittel bei Raumtemperatur vermischt und in einem Stahlwerkzeug zu 4 mm dicken ebenen Formteilen ausgehärtet. Aus diesen Formteilen werden dann durch Sägen bzw. Fräsen Probekörper entnommen, an denen unter Einhaltung der nachstehend genannten Prüfnormen die in Tabelle 2 aufgeführten Eigenschaftswerte ermittelt sind.

| | | Prüfkörperabmessungen |
|---|---|---|
| Biegefestigkeit | DIN 53 482 | 80 x 10 x 4 mm³ |
| Durchbiegung | DIN 53 452 | 80 x 10 x 4 mm³ |
| Schlagzähigkeit | DIN 53 453 | 50 x 6 x 4 mm³ |
| Zugfestigkeit | DIN 53 455 | Schulterstab Nr. 3 |
| Dehnung | DIN 53 455 | Schulterstab Nr. 3 |
| Elastizitätsmodul | DIN 53 457 | Schulterstab Nr. 3 |
| Martenstemperatur | DIN 53 458 | 60 x 15 x 4 mm³ |
| Heat Distortion Temperature (HDT) | DIN 53 461 | 120 x 10 x 4 mm³ |
| Glasübergangstemperatur (TG) | DIN 53 445 | 80 x 10 x 1 mm³ |

**Tabelle 2**

| Eigenschaften der unverstärkten Formstoffe | | |
|---|---|---|
| | Härtungsmittel Beispiel 1 | Beispiel 6 |
| Härtemenge g | 14 | 14 |
| Härtebedingungen | 2 h 120 °C | 2 h 120 °C |
| Biegefestigkeit N/mm² | 90 | 41 |
| Durchbiegung mm | 7 | 11 |
| Schlagzähigkeit kJ/m² | 6 | 20 |
| Zugfestigkeit N/mm² | 50 | 30 |
| Dehnung % | 2,0 | 2,0 |
| Elastizitätsmodul N/mm² | 3000 | 3000 |
| Martenstemperatur °C | 113 | 107 |
| HDT °C | 130 | 129 |
| TG °C | 156 | 158 |

## Patentansprüche

1. Amid- und gegebenenfalls Amingruppen enthaltende Verbindungen auf Basis von Imidazol- und/oder N-Alkylimidazol-Acrylsäure-Reaktionsprodukten, herstellbar in erster Stufe durch
A) Addition von Acrylsäure(estern) an gegebenenfalls substituierte Imidazole und/oder N-Aminoalkylimidazole in ca. equivalenten Mengen, bezogen auf reaktive Aminwasserstoffatome und Doppelbindungen und
weitere Umsetzung dieser Addukte in zweiter Stufe mit
B) Polyaminen und/oder Aminoalkoholen, welche im Durchschnitt zwei oder mehr reaktive Wasserstoffatome im Molekül haben, unter Kondensationsbedingungen zu Polyamiden und/oder Polyaminoamiden bzw. Polyamidoalkoholen, wobei
gegebenenfalls in einer dritten Stufe
C) die Reaktionsprodukte AB) mit ca. equivalenten Mengen an Mono- und/oder Polyglycidylverbindungen, Mono- und/ oder Polyacrylaten, Mono- und/oder Polycarbonsäuren verlängert werden.

2. Verbindungen gemäß Anspruch 1, dadurch gekennzeichnet, daß in erster Stufe Acrylsäure mit Imidazol und/oder 2-Methylimidazol und/oder N-Aminopropylimidazol umgesetzt wird und die weitere Umsetzung dieser Addukte in zweiter Stufe mit Diglykolamin und/oder Diethylentriamin und/oder N-Aminoethylpiperazin erfolgt.

3. Verbindungen gemäß den Ansprüchen 1 und 2, dadurch gekennzeichnet, daß die Additionsprodukte der Stufen A) + B) in dritter Stufe C) mit mindestens einer der nachfolgenden Verbindungen Butandioldiglycidylether, Hexandioldiglycidylether, Butandioldiacrylat, Hexandioldiacrylat, dimerisierte Fettsäure durchgeführt wird.

4. Verwendung der Verbindungen gemäß den Ansprüchen 1 bis 3 als Härtungsmittel für Epoxidharze.

5. Härtbare Epoxidharzzusammensetzung, enthaltend
a) ein Epoxidharz mit durchschnittlich mehr als einer Epoxidgruppe pro Molekül und
b) mindestens eine der Verbindungen gemäß den Ansprüchen 1 bis 3

6. Härtbare Epoxidharzzusammensetzungen, worin die Verstärkungs- und Einlagematerialien bei Raumtemperatur mit dem Bindemittel, bestehend aus
a) einem Epoxidharz mit im Durchschnitt mehr als einer Epoxidgruppe pro Molekül und
b) mindestens einer der gemäß den Ansprüchen 1 bis 3 herstellbaren Verbindungen und gegebenenfalls
c) Lösungsmitteln, Füllstoffen, Pigmenten, Hilfsstoffen getränkt und gegebenenfalls bei erhöhter Temperatur in den halbfesten aber noch schmelzbaren Zustand (B-Zustand) überführt werden.

7. Epoxidharzformkörper, dadurch gekennzeichnet, daß in erster Stufe die Verstärkungs- und Einlagematerialien bei Raumtemperatur mit dem Bindemittel, bestehend aus
a) einem Epoxidharz mit im Durchschnitt mehr als einer Epoxidgruppe pro Molekül und
b) mindestens einer der gemäß den Ansprüchen 1 bis 3 herstellbaren Verbindungen und gegebenenfalls
c) Lösungsmitteln, Füllstoffen, Pigmenten, Hilfsstoffen
überführt werden und in zweiter Stufe die Laminate oder Prepregs unter Formgebung oder zwischen zu verklebende Substrate gebracht, unter Anwendung von Druck bei erhöhter Temperatur ausgehärtet werden.

8. Verfahren zur Herstellung von faserverstärkten Trägermaterialien für den Elektrosektor durch Überführung von mit Bindemittel auf Basis von Epoxidharzen und aminischen Härtungsmitteln imprägnierten Verstärkungsmaterialien in erster Stufe durch Anwendung von Temperatur und gegebenenfalls von Druck in den B-Zustand überführt und in zweiter Stufe bei erhöhter Temperatur ausgehärtet werden, dadurch gekennzeichnet, daß als Epoxidharze
a) bromierte Epoxidharze mit im Durchschnitt mehr als einer Epoxidgruppe pro Molekül
und als aminische Härtungsmittel
b) mindestens eine der Verbindungen gemäß den Ansprüchen 1 bis 3 verwendet werden.
